# FASCICULE DE BREVET EUROPEEN

(11) **EP 0 855 898 B1**
(45) Date de publication et mention de la délivrance du brevet: **05.09.2001**
(21) Numéro de dépôt: 96934946.3
(22) Date de dépôt: 21.10.1996
(51) Int. Cl.: A61K 7/06, A61K 7/09

(54) **NOUVELLE COMPOSITION REDUCTRICE ET NOUVEAU PROCEDE POUR LA DEFORMATION PERMANENTE DES CHEVEUX**
NEUE REDUZIERENDE ZUSAMMENSETZUNG UND EIN NEUES VERFAHREN ZUR DAUERHAFTEN HAARVERFORMUNG
NOVEL REDUCING COMPOSITION AND NOVEL METHOD FOR PERMING HAIR

(30) Priorité: 20.10.1995 FR 9512399
(43) Date de publication de la demande: 05.08.1998
(73) Titulaire: L'OREAL, 75008 Paris (FR)
(72) Inventeur: MAUBRU, Mireille, F-78400 Chatou (FR); BRAIDA-VALERIO, Damarys, F-75004 Paris (FR)
(74) Mandataire: Andral, Christophe André Louis
(86) Numéro de dépôt international: FR9601643
(87) Numéro de publication internationale: WO9715272

(56) Documents cités:
- EP-A- 0 647 617
- FR-A- 2 679 770

## Description

L'invention concerne une composition cosmétique pour le premier temps d'un procédé de déformation permanente des fibres kératiniques et un procédé de traitement des matières kératiniques, en particulier des cheveux, en vue d'obtenir une déformation permanente de ces dernières, en particulier sous la forme de cheveux permanentés.

On sait que la technique la plus usuelle pour obtenir une déformation permanente des cheveux consiste, dans un premier temps, à réaliser l'ouverture des liaisons disulfures -S-S- de la kératine (cystine) à l'aide d'une composition contenant un agent réducteur adapté (étape de réduction) puis, après avoir rincé la chevelure ainsi traitée, à reconstituer dans un second temps lesdites liaisons disulfures en appliquant, sur les cheveux préalablement mis sous tension (bigoudis et autres), une composition oxydante (étape d'oxydation, dite aussi de fixation) de façon à donner finalement aux cheveux la forme recherchée. Cette technique permet ainsi de réaliser indifféremment soit l'ondulation des cheveux, soit leur défrisage ou leur décrêpage. La nouvelle forme imposée aux cheveux par un traitement chimique tel que ci-dessus est éminemment durable dans le temps et résiste notamment à l'action des lavages à l'eau ou par shampooings, et ceci par opposition aux simples techniques classiques de déformation temporaire, telles que de mise en pli.

Les compositions réductrices utilisables pour la mise en oeuvre de la première étape d'une opération de permanente contiennent généralement, à titre d'agents réducteurs, des sulfites, des bisulfites, des alkyl-phosphines ou de préférence des thiols.

Concernant les compositions oxydantes nécessaires à la mise en oeuvre de l'étape de fixation, on fait le plus souvent appel, dans la pratique, à des compositions à base d'eau oxygénée ou de bromates alcalins.

Le problème de la technique des permanentes connues à ce jour est que leur application sur les cheveux induit à la longue une altération de la qualité de ces derniers. Les causes essentielles de cette altération de la qualité des cheveux sont une diminution de leurs propriétés cosmétiques, telles que leur brillance, et une dégradation de leurs propriétés mécaniques, plus particulièrement une dégradation de leur résistance mécanique due à un gonflement des fibres kératiniques lors du rinçage entre l'étape de réduction et l'étape d'oxydation qui peut également se traduire par une augmentation de leur porosité.

Les cheveux sont affaiblis et peuvent devenir cassants lors de traitements ultérieurs comme des brushings.

Pour résoudre ce problème d'altération de la qualité des cheveux, il a été proposé d'associer des polymères cationiques soit aux agents réducteurs, soit aux agents oxydants.

Mais ces solutions se révèlent insatisfaisantes dans la mesure où elles ne résolvent pas totalement le problème de la diminution des propriétés mécaniques des cheveux. En particulier, dans le cas d'un traitement de déformation permanente des cheveux, ces derniers présentent un toucher non satisfaisant et la tenue de la frisure est insuffisante.

La présente invention a notamment pour but de résoudre les problèmes ci-dessus.

Plus précisément, elle a pour but de proposer une nouvelle composition réductrice stable qui, utilisée notamment lors du premier temps d'une opération de déformation permanente des fibres kératiniques permet de limiter, voire d'empêcher, la dégradation des propriétés mécaniques de ces dernières, et plus particulièrement la casse des cheveux, et d'obtenir ainsi une belle frisure résistante au brushing et de bonne tenue.

Elle a également pour but de proposer une composition réductrice telle que ci-dessus qui permette d'améliorer les propriétés cosmétiques, telles que la douceur et la facilité de démêlage, des fibres kératiniques lorsque celles-ci subissent en particulier un traitement de déformation permanente.

Enfin, la présente invention a pour but de proposer un nouveau procédé de déformation permanente des fibres kératiniques, en particulier des cheveux, utilisant une composition réductrice selon l'invention.

Il a été proposé dans les demandes EP-A-0 647 617 et FR A 2 673 179 au nom de la Demanderesse d'utiliser des céramides particuliers en association avec des lipides comme enveloppe de vésicules encapsulant des substances actives hydrosolubles, ces substances actives pouvant être, entre autres, des réducteurs, pour protéger lesdites substances actives des différents agents d'altération et des composés réactifs qui peuvent être présents dans la composition.

Or, la demanderesse vient de découvrir de façon tout à fait surprenante que l'utilisation de composés de type céramide dans une composition réductrice d'un procédé de déformation permanente exempte de vésicules encapsulant un agent réducteur permettait d'obtenir un excellent état de la fibre capillaire au terme du procédé de permanente.

La présente invention a donc pour objet une nouvelle composition, pour le premier temps d'un procédé de déformation permanente des fibres kératiniques, en particulier des cheveux, caractérisée par le fait qu'elle comprend, dans un support cosmétique approprié, i) au moins un composé de type céramide et ii) au moins un agent réducteur répondant à la formule (I) suivante : 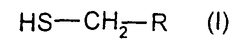 où R désigne un radical carboxy, aminoalkyle en C₁-C₄, carboxyalkyle en C₁-C₄ éventuellement substitué par un groupement amino, uréidoalkyle en C₁-C₄, acyle en C₁-C₄ aminoalkyle en C₁-C₄, aminoacyle en C₁-C₄ aminoalkyle en C₁-C₄, alcoxy en C₁-C₄ carbonyle, monohydroxyalcoxy en C₁-C₄ carbonyle, dihydroxyalcoxy en C₂-C₄ carbonyle, alcoxy en C₁-C₄ carbonyle alkyle en C₁-C₄ éventuellement substitué par un groupement amino, monohydroxy alcoxy en C₁-C₄ carbonyle alkyle en C₁-C₄ éventuellement substitué par un groupement amino, ou dihydroxyalcoxy en C₂-C₄ carbonylalkyle en C₁-C₄ éventuellement substitué par un groupement amino,
ledit composé de type céramide étant présent dans la composition à une teneur allant de 0,005 % à 10 % en poids par rapport au poids total de la composition, ladite composition étant exempte de vésicules contenant un agent réducteur.

La présente invention a également pour objet un nouveau procédé de traitement des matières kératiniques, en particulier des cheveux, en vue d'obtenir une déformation permanente de ces dernières, en particulier sous la forme de cheveux permanentés, ce procédé comprenant les étapes suivantes : (i) on applique sur la matière kératinique à traiter une composition telle que définie ci-dessus, la matière kératinique étant mise sous tension mécanique avant, pendant, ou après ladite application, (ii) on rince éventuellement la matière kératinique, (iii) on applique sur la matière kératinique une composition oxydante ou on laisse reposer la matière kératinique éventuellement sous chaleur, (iv) on rince éventuellement à nouveau la matière kératinique.

Le procédé selon l'invention convient particulièrement bien à l'obtention d'une chevelure permanentée sans risque de dégradation de la fibre kératinique. En particulier, le procédé selon l'invention limite la casse des cheveux. On obtient une belle frisure homogène, ainsi qu'une meilleure tenue de la coiffure. Le toucher mouillé des cheveux traités selon le procédé de l'invention est agréable et le coiffage est facilité. La forme aquise par des cheveux ayant subi le traitement de déformation permanente selon l'invention présente en outre une bonne rémanence dans le temps au shampooing.

D'autres caractéristiques, aspects et avantages de l'invention apparaîtront encore plus clairement à la lecture de la description détaillée qui va suivre, ainsi que des divers exemples concrets, mais nullement limitatifs, destinés à l'illustrer.

Bien que l'exposé qui suit s'articule essentiellement autour du cas particulier du traitement du cheveu, on notera ici que le procédé selon l'invention est applicable à toute matière kératinique en général, notamment cils, moustaches, poils, laine et autres.

Dans ce qui précède et ce qui suit, on entend par vésicules des sphérules lipidiques constituées de couches moléculaires organisées enfermant une phase aqueuse encapsulée, ces couches étant constituées d'au moins un composé de type céramide associé à au moins un autre composé lipidique.

Selon la présente invention, on entend, par composé de type céramide, les céramides et/ou les glycocéramides et/ou les pseudocéramides. Ils sont choisis de préférence parmi les molécules naturelles ou synthétiques répondant à la formule (II) suivante : 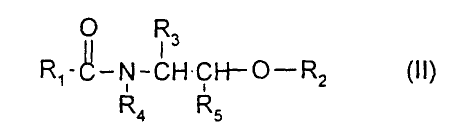 dans laquelle :
- R₁ désigne :
   - soit un radical hydrocarboné, linéaire ou ramifié, saturé ou insaturé, en C₁-C₅₀, de préférence en C₅-C₅₀, ce radical pouvant être substitué par un ou plusieurs groupements hydroxyle éventuellement estérifiés par un acide R₇COOH, R₇ étant un radical hydrocarboné, saturé ou insaturé, linéaire ou ramifié, éventuellement mono ou polyhydroxylé, en C₁-C₃₅, le ou les hydroxyles du radical R₇ pouvant être estérifiés par un acide gras saturé ou insaturé, linéaire ou ramifié, éventuellement mono ou polyhydroxylé, en C₁-C₃₅,
   - soit un radical R"-(NR-CO)-R', R désigne un atome d'hydrogène ou un radical hydrocarboné C₁-C₂₀ mono ou polyhydroxylé, préférentiellement monohydroxylé, R' et R" sont des radicaux hydrocarbonés dont la somme des atomes de carbone est comprise entre 9 et 30, R' étant un radical divalent,
   - soit un radical R₈-O-CO-(CH2)ₚ, R₈ désignant un radical hydrocarboné en C₁-C₂₀, p étant un entier variant de 1 à 12 ;
- R₂ est choisi parmi un atome d'hydrogène, un radical de type saccharidique, en particulier un radical (glycosyle)ₙ, (galactosyle)ₘ ou sulfogalactosyle, un résidu de sulfate ou de phosphate, un radical phosphoryléthylamine et un radical phosphoryléthylammonium, dans lesquels n est un entier variant de 1 à 4 et m est un entier variant de 1 à 8 ;
- R₃ désigne un atome d'hydrogène ou un radical hydrocarboné en C₁-C₃₃, saturé ou insaturé, hydroxylé ou non, le ou les hydroxyles pouvant être estérifiés par un acide minéral ou un acide R₇COOH, R₇ ayant les mêmes significations que ci-dessus, le ou les hydroxyles pouvant être éthérifiés par un radical (glycosyle)ₙ, (galactosyle)_{m,} sulfogalactosyle, phosphoryléthylamine ou phosphoryléthylammonium, R₃ pouvant également être substitué par un ou plusieurs radicaux alkyle en C₁-C₁₄ ;
   de préférence, R₃ désigne un radical α-hydroxyalkyle en C₁₅-C₂₆, le groupement hydroxyle étant éventuellement estérifié par un α-hydroxyacide en C₁₆-C₃₀ ;
- R₄ désigne un atome d'hydrogène, un radical méthyle, éthyle, un radical hydrocarboné en C₃-C₅₀, saturé ou insaturé, linéaire ou ramifié, éventuellement hydroxylé ou un radical -CH₂-CHOH-CH₂-O-R₆ dans lequel R₆ désigne un radical hydrocarboné en C₁₀-C₂₆ ou un radical R₈-O-CO-(CH2)ₚ, R₈ désigne un radical hydrocarboné en C₁-C₂₀, p étant un entier variant de 1 à 12,
- R₅ désigne un atome d'hydrogène ou un radical hydrocarboné en C₁-C₃₀ saturé ou insaturé, linéaire ou ramifié, éventuellement mono ou polyhydroxylé, le ou les hydroxyles pouvant être éthérifiés par un radical (glycosyle)ₙ, (galactosyle)ₘ, sulfogalactosyle, phosphoryléthylamine ou phosphoryléthylammonium,
sous réserve que lorsque R₃ et R₅ désignent hydrogène ou lorsque R₃ désigne hydrogène et R₅ désigne méthyle alors R₄ ne désigne pas un atome d'hydrogène, un radical méthyle ou éthyle.

Parmi les composés de formule (II) ci-dessus, on préfère les céramides et/ou glycocéramides décrits par DOWNING dans Journal of Lipid Research, Vol. 35, page 2060, 1994 ou ceux décrits dans la demande de brevet français FR-2 673 179, et dont les enseignements sont ici inclus à titre de référence.

Les composés de type céramide plus particulièrement préférés selon l'invention sont les composés de formule (II) pour lesquels R₁ désigne un radical alkyle saturé ou insaturé dérivé diacides gras en C₁₄-C₂₂ éventuellement hydroxylé; R₂ désigne un atome d'hydrogène ; et R₃ désigne un radical linéaire saturé en C₁₁-C₁₇ éventuellement hydroxylé et de préférence en C₁₃-C₁₅.

De tels composés sont par exemple :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol
ou les mélanges de ces composés.

On peut aussi utiliser des mélanges spécifiques tels que par exemple les mélanges de céramide(s) 2 et de céramide(s) 5 selon la classification de DOWNING.

On peut également utiliser les composés de formule (II) pour lesquels R₁ désigne un radical alkyle saturé ou insaturé dérivé d'acides gras ; R₂ désigne un radical galactosyle ou sulfogalactosyle ; et R₃ désigne un radical hydrocarboné en C₁₂-C₂₂, saturé ou insaturé et de préférence un groupement -CH=CH-(CH₂)₁₂-CH₃.

Des composés de type céramide sont par exemple décrits dans les demandes de brevet DE4424530, DE4424533, DE4402929, DE4420736, WO95/23807, WO94/07844, EP-A-0646572, WO95/16665, FR-2 673 179, EP-A-0227994 et WO 94/07844, WO94/24097, WO94/10131 dont les enseignements sont ici inclus à titre de référence.

A titre d'exemple, on peut citer le produit constitué d'un mélange de glycocéramides, vendu sous la dénomination commerciale GLYCOCER par la société WAITAKI INTERNATIONAL BIOSCIENCES.

On peut également utiliser les composés décrits dans les demandes de brevet EP-A-0 227 994, EP-A-0 647 617, EP-A-0 736 522 et WO 94 / 07 844.

De tels composés sont par exemple le QUESTAMIDE H, encore appelé bis-(N-hydroxyéthyl N-cétyl) malonamide et vendu par la société QUEST et le N-(2-hydroxyéthyl)-N-(3-cétyloxy-2-hydroxypropyl)amide d'acide cétylique .

On peut également utiliser le N-docosanoyl N-méthyl-D-glucamine tel que décrit dans la demande de brevet WO 94 / 24097.

De préférence, le composé de type céramide utilisé dans la présente invention est choisi parmi le 2-N-oléoylamino-octadécane-1,3-diol, le 2-N-[2-hydroxy-palmitoyl]-amino-octadécane-1,3-diol et la N-stéaroylphytosphingosine.

Le ou les composés de type céramide sont présents dans la composition selon l'invention à une teneur allant de 0,005 % à 10 %, de préférence de 0,006 % à 10 %, et de préférence encore de 0,008 % à 4 %.

L'agent réducteur de la composition selon l'invention est choisi parmi les composés répondant à la formule (I) suivante : 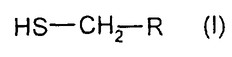 où R désigne un radical carboxy, aminoalkyle en C₁-C₄, carboxyalkyle en C₁-C₄ éventuellement substitué par un groupement amino, uréidoalkyle en C₁-C₄, acyle en C₁-C₄ aminoalkyle en C₁-C₄, aminoacyle en C₁-C₄ aminoalkyle en C₁-C₄, alcoxy en C₁-C₄ carbonyle, monohydroxyalcoxy en C₁-C₄ carbonyle, dihydroxyalcoxy en C₂-C₄ carbonyle, alcoxy en C₁-C₄ carbonyle alkyle en C₁-C₄ éventuellement substitué par un groupement amino, monohydroxy alcoxy en C₁-C₄ carbonyle alkyle en C₁-C₄ éventuellement substitué par un groupement amino, ou dihydroxyalcoxy en C₂-C₄ carbonylalkyle en C₁-C₄ éventuellement substitué par un groupement amino.

En particulier, R est choisi parmi les radicaux suivants : -CO₂H ; -CH₂-NH₂ ; -CH(NH₂)-CO₂H ; -(CH₂)₂-CO₂H ; -CH₂-CO₂H ; -CH₂-NH-CONH₂ ; -CH₂-NH-COCH₃ ; -CH₂-NH-CO-CH₂CH₃ ; -CH₂-NH-CO(CH₂)₂-NH₂ ; -CH₂-NH-CO-CH₂-NH₂; -CH₂-NH-CO-CH(CH₃)-NH₂ ; -(CO)O-CH₂-CH(OH)-CH₂OH ; -CH(NH₂)-COOR' où R' est un radical alkyle en C1-C4 mono- ou dihydroxylé.

De préférence, l'agent réducteur de la composition selon l'invention est choisi dans le groupe formé par l'acide thioglycolique, la cystéamine et la cystéine. Encore plus préférentiellement, l'agent réducteur des compositions selon l'invention est l'acide thioglycolique.

L'agent réducteur est généralement présent dans la composition selon l'invention à une teneur allant de 1 % à 25 %, de préférence allant de 3 % à 15 %.

Le pH de l'ensemble de la composition réductrice est de préférence compris entre 5 et 11 et encore plus préférentiellement entre 6,5 et 10.

Ce pH peut être obtenu et/ou ajusté classiquement par ajout soit d'agents basifiants, tels que par exemple l'ammoniaque, la monoéthanolamine, la diéthanolamine, la triéthanolamine, l'isopropanolamine, la propanediamine-1,3, un carbonate ou bicarbonate alcalin ou d'ammonium, un carbonate organique tel que le carbonate de guanidine, ou bien encore un hydroxyde alcalin, tous ces composés pouvant bien entendu être pris seuls ou en mélange, soit d'agents acidifiants tels que par exemple l'acide chlorhydrique, l'acide acétique, l'acide lactique ou l'acide borique.

La composition réductrice peut se présenter sous la forme d'une lotion, épaissie ou non, d'une crème, d'un gel ou de toute autre forme appropriée et peut contenir des additifs connus pour leur utilisation dans les compositions réductrices pour la déformation permanente des cheveux.

La composition réductrice peut être également du type exothermique, c'est-à-dire provoquant un certain échauffement lors de l'application sur les cheveux, ce qui apporte un agrément à la personne qui subit la permanente ou le défrisage.

La composition réductrice peut également contenir un solvant tel que par exemple de l'éthanol, du propanol, ou de l'isopropanol ou encore du glycérol à une concentration maximale de 20 % par rapport au poids total de la composition.

Lorsque les compositions sont destinées à une opération de défrisage ou de décrêpage des cheveux, la composition réductrice est de préférence sous forme d'une crème épaissie de façon à maintenir les cheveux aussi raides que possible. On réalise ces crèmes, sous forme d'émulsions "lourdes", par exemple à base de stéarate de glycéryle, de stéarate de glycol, de cires auto-émulsionnables, d'alcools gras, etc.

On peut également utiliser des liquides ou des gels contenant des agents épaississants tels que des polymères ou des copolymères carboxyvinyliques qui "collent" les cheveux et les maintiennent dans la position lisse pendant le temps de pose.

Enfin, les compositions peuvent être également sous forme dite "auto-neutralisante" ou encore "auto-régulée" et dans ce cas, les agents réducteurs utilisés selon l'invention sont associés à au moins un disulfure connu pour son utilisation dans une composition réductrice pour permanente auto-neutralisante.

Parmi de tels disulfures connus, on peut notamment mentionner l'acide dithioglycolique, le dithioglycérol, la cystamine, la N, N'-diacétyl-cystamine, la cystine, la pantéthine, et les disulfures des N-(mercapto-alkyl) ω-hydroxyalkyl-amides décrits dans la demande de brevet EP-A-354 835, les disulfures des N-mono ou N,N-dialkylmercapto-4 butyramides décrits dans la demande de brevet EP-A-368 763, les disulfures des aminomercapto-alkylamides décrits dans la demande de brevet EP-A-432 000, les disulfures des dérivés des acides N-(mercaptoalkyl)-succinamiques ou des N-(mercaptoalkyl)-succinimides décrits dans la demande de brevet EP-A-465 342, les disulfures des alkylaminomercaptoalkylamides décrits dans la demande de brevet EP-A-514 282 et les disulfures des N-mercaptoalkyl alcane diamides décrits dans la demande de brevet EP-A-653.202. Ces disulfures sont généralement présents dans un rapport molaire de 0,5 à 2,5, et de préférence de 1 à 2, par rapport à l'agent réducteur (voir brevet US 3 768 490).

Un deuxième objet de la présente invention est un procédé de traitement des fibres kératiniques utilisant comme composition réductrice la composition définie ci-dessus. L'application de cette composition sur les cheveux constitue généralement la première étape de ce procédé. Celle-ci se fait mèche par mèche ou globalement.

L'habituelle étape de mise sous tension des cheveux sous une forme correspondant à la forme finale désirée pour ces derniers (boucles par exemple) peut être mise en oeuvre par tout moyen, mécanique notamment, approprié et connu en soi pour maintenir sous tension des cheveux, tels que par exemple rouleaux, bigoudis et analogues.

Les cheveux peuvent également être mis en forme sans l'aide de moyens extérieurs, simplement avec les doigts.

Avant de procéder à l'étape suivante facultative de rinçage, il convient, de manière classique, de laisser reposer pendant quelques minutes, généralement entre 10 minutes et une heure, de préférence entre 20 et 40 minutes, la chevelure sur laquelle a été appliquée la composition réductrice, et ceci de façon à bien laisser le temps au réducteur d'agir correctement sur les cheveux. Cette phase d'attente est effectuée de préférence à une température allant de 35 °C à 45 °C, en protégeant de préférence également les cheveux par un bonnet.

Dans la deuxième étape, facultative, du procédé (étape (ii)), les cheveux imprégnés de la composition réductrice sont donc ensuite rincés soigneusement par une composition aqueuse.

Puis, dans une troisième étape (étape (iii)), facultative également, on applique sur les cheveux ainsi rincés une composition oxydante, ou fixateur, dans le but de fixer la nouvelle forme imposée aux cheveux. Cette troisième étape peut également être une étape de repos de la matière kératinique, éventuellement sous chaleur.

La composition oxydante contient un agent oxydant qui peut être choisi parmi l'eau oxygénée, un bromate alcalin, un persel ou un polythionate ou leur mélange, tel qu'un mélange de bromate alcalin et d'un persel.

Ce fixateur peut également se trouver sous la forme d'un shampooing.

La composition oxydante peut contenir en outre des additifs cosmétiques bien connus pour ce type de composition tels que des agents alcalinisants ou acidifiants, des agents conservateurs, des agents séquestrants, des cations, des opacifiants et éventuellement un polymère cationique.

La composition oxydante peut également contenir un céramide et/ou glycocéramide tel que défini ci-dessus.

Comme dans le cas de l'application de la composition réductrice, la chevelure sur laquelle a été appliquée la composition oxydante est ensuite, de manière classique, laissée dans une phase de repos ou d'attente qui dure quelques minutes, généralement entre 3 et 30 minutes, de préférence entre 5 et 15 minutes.

Le véhicule des compositions réductrice et oxydante utilisées selon l'invention est de préférence l'eau ou une solution hydroalcoolique d'un alcool inférieur tel que l'éthanol, l'isopropanol ou le butanol.

L'eau oxygénée peut être stabilisée par exemple par la phénacétine, l'acétanilide, les phosphates mono et trisodiques ou par le sulfate d'hydroxy-8 quinoléine.

Si la tension des cheveux était maintenue par des moyens extérieurs, on peut retirer de la chevelure ces derniers (rouleaux, bigoudis et analogues) avant ou après l'étape de fixation.

Enfin, dans la dernière étape du procédé selon l'invention (étape (iv)), étape facultative également, les cheveux imprégnés de la composition oxydante sont rincés soigneusement, généralement à l'eau.

On obtient finalement une chevelure facile à démêler, douce. Les cheveux sont ondulés.

Des exemples concrets illustrant l'invention vont maintenant être donnés.

Dans ce qui suit ou ce qui précède, sauf mention contraire, les pourcentages sont exprimés en poids.

### EXEMPLE 1 :

La demanderesse a réalisé un test comparatif afin de mettre en évidence l'amélioration apportée au niveau de la résistance mécanique des fibres kératiniques par l'adjonction de céramides dans une composition réductrice utilisée dans un procédé de traitement pour la déformation permanente des fibres kératiniques.

On a réalisé la composition réductrice A, conforme à l'invention, suivante :

### Réducteur A :

- 2-N-oléoylamino-octadécane-1,3-diol (céramide) 1 %
- mélange cocoylamidopropyl bétaïne/monolaurate de glycérol à 30 % MA 2 %
- acide thioglycolique 6,7 %
- bicarbonate d'ammonium 5,1 %
- séquestrant 0,2 %
- ammoniaque à 20 % NH₃ 6,2 %
- eau déminéralisée qsp 100 %

On a également réalisé une composition réductrice B, comparative, de même composition que A mais ne contenant pas de N-oléyldihydrosphingosine.

Les compositions réductrices ci-dessus ont été réalisées par simple mélange, après dissolution ou dispersion et chauffage du céramide.

Afin de comparer les deux compositions réductrices lors d'un traitement de déformation permanente des cheveux, on a réalisé une composition oxydante, ou fixateur, de composition suivante :

### Fixateur :

- peroxyde d'hydrogène à 200 volumes 4,8 %
- oxyde de lauryl diméthyl amine en solution aqueuse à 30 % MA 2,15 % en l'état
- acide citrique qsp pH=3
- eau déminéralisée qsp 100 %

La composition oxydante a été réalisée par simple mélange.

On a appliqué chacune des compositions A et B sur des mèches de cheveux sensibilisés, avec un rapport de bain de 2 g / g de cheveux. Par cheveux sensibilisés, on entend des cheveux abîmés à des degrés divers par l'action des agents atmosphériques et/ou de traitements capillaires, mécaniques ou chimiques, tels que des colorations, des décolorations et/ou des permanentes. Après 10 minutes de pose, un rinçage à l'eau a été effectué.

On a ensuite appliqué le fixateur sur les cheveux rincés, avec un rapport de bain de 2 g / g de cheveux. Après 5 minutes de pose, les cheveux ont été rincés puis séchés.

L'aptitude de chaque composition à limiter la dégradation de la fibre kératinique a été évaluée selon le protocole suivant : pour chaque chevelure préalablement traitée de la façon indiquée ci-dessus avec la composition A ou B, on a mouillé trois mèches de cheveux qu'on a ensuite disposées sur un support métallique, afin de maintenir les cheveux à la racine. On a ensuite réalisé un brushing de la manière la plus régulière possible au moyen d'une brosse à brushing de marque déposée « Babyliss » ayant déjà servi à plus de 50 brushings.

Les cheveux cassés lors du brushing ont été minutieusement récupérés sur la brosse, introduits dans une boîte de Pétri puis pesés après un conditionnement de 12 heures à une humidité relative de 50% ± 2% et à une température de 20 °C ± 2°C.

Les résultats obtenus sont consignés dans le tableau (I) ci-dessous :

**Tableau (I) :**

| Formule | Quantité de cheveux cassés mg/g |
|---|---|
| Composition A (invention) | 14, 7 ± 1,3 |
| Composition B (comparatif) | 30,3 ± 2,6 |

Ces résultats montrent clairement que l'introduction d'un céramide dans une composition réductrice d'un procédé de déformation permanente limite fortement la dégradation de la fibre kératinique.

### EXEMPLE 2 :

Un exemple concret de composition réductrice pour un procédé de déformation permanente des cheveux est donné ci-après :
- 2-N-oléoylamino-octadécane-1,3-diol 0,01 %
- cocoyl bétaïne 2 %
- acide thioglycolique 7 %
- bicarbonate d'ammonium 5,5 %
- ammoniaque à 20% NH₃ 6,6 %
- séquestrant 0,4 %
- eau déminéralisée qsp 100 %

### EXEMPLE 3 :

Un exemple concret de composition réductrice pour un procédé de déformation permanente des cheveux est donné ci-après :
- N 2 hydroxy hexadécanoyl 2 amino octadécane 1,3 diol 0,01 %
- L-cystéine 5,5 %
- cocoylbétaïne 1 %
- monoéthanolamine 6 %
- séquestrant 0,4 %
- eau déminéralisée qsp 100 %

### EXEMPLE 4 :

Un exemple concret de composition réductrice pour un procédé de déformation permanente des cheveux est donné ci-après :
- 2-N-oléoylamino-octadécane-1,3-diol 0,15 %
- ammoniaque à 20% NH₃ 4,9 %
- chlorhydrate de cystéamine 9,5 %
- séquestrant 0,4 %
- eau déminéralisée qsp 100 %

### EXEMPLE 5 :

La demanderesse a réalisé un test comparatif afin de mettre en évidence l'importance de la teneur en composé de type céramide dans les compositions réductrices selon l'invention, utilisées dans un procédé de traitement pour la déformation permanente des fibres kératiniques.

On a réalisé la composition réductrice C, conforme à l'invention, suivante :

### Réducteur C :

- 2-N-oléoylamino-octadécane-1,3-diol (céramide) 0,006 %
- mélange cocoylamidopropyl bétaïne/monolaurate de glycérol à 30 % MA 2 % MA
- acide thioglycolique 6,7 %
- bicarbonate d'ammonium 5,1 %
- séquestrant 0,2 %
- ammoniaque à 20 % NH₃ 6,2 %
- eau déminéralisée qsp 100 %

On a également réalisé une composition réductrice D, comparative, de même composition que C, contenant également de la N-oléyldihydrosphingosine mais dans une proportion ne rentrant pas dans le cadre de l'invention.

### Réducteur D :

- 2-N-oléoylamino-octadécane-1,3-diol (céramide) 0,0025 %
- mélange cocoylamidopropyl bétaïne/monolaurate de glycérol à 30 % MA 2 % MA
- acide thioglycolique 6,7 %
- bicarbonate d'ammonium 5,1 %
- séquestrant 0,2 %
- ammoniaque à 20 % NH₃ 6,2 %
- eau déminéralisée qsp 100 %

Les compositions réductrices ci-dessus ont été réalisées par simple mélange, après dissolution ou dispersion et chauffage du céramide.

Afin de comparer les deux compositions réductrices lors d'un traitement de déformation permanente des cheveux, on a réalisé une composition oxydante, ou fixateur, de composition suivante :

### Fixateur

- peroxyde d'hydrogène à 200 volumes 4,8 %
- oxyde de lauryl diméthyl amine en solution aqueuse à 30 % MA 2,15% en l'état
- acide citrique qsp pH=3
- eau déminéralisée qsp 100 %

La composition oxydante a été réalisée par simple mélange.

On a appliqué chacune des compositions C et D sur des mèches de cheveux sensibilisés, avec un rapport de bain de 2 g / g de cheveux. Par cheveux sensibilisés, on entend des cheveux abîmés à des degrés divers par l'action des agents atmosphériques et/ou de traitements capillaires, mécaniques ou chimiques, tels que des colorations, des décolorations et/ou des permanentes. Après 10 minutes de pose, un rinçage à l'eau a été effectué.

On a ensuite appliqué le fixateur sur les cheveux rincés, avec un rapport de bain de 2 g / g de cheveux. Après 5 minutes de pose, les cheveux ont été rincés puis séchés.

L'aptitude de chaque composition à limiter la dégradation de la fibre kératinique a été évaluée selon le protocole suivant : pour chaque chevelure préalablement traitée de la façon indiquée ci-dessus avec la composition C ou D, on a mouillé quatre mèches de cheveux qu'on a ensuite disposées sur un support métallique, afin de maintenir les cheveux à la racine. On a ensuite réalisé un brushing de la manière la plus régulière possible au moyen d'une brosse à brushing de marque déposée « Delorme » ayant servi à moins de 4 brushings.

Les cheveux cassés lors du brushing ont été minutieusement récupérés sur la brosse, introduits dans une boîte de Pétri puis pesés après un conditionnement de 12 heures à une humidité relative de 50% ± 2% et à une température de 20 °C ± 2°C.

Les résultats obtenus sont consignés dans le tableau (II) ci-dessous :

**Tableau (II) :**

| Formule | Quantité de cheveux cassés mg/g |
|---|---|
| Composition C (invention) | 55 ± 9 |
| Composition D (comparatif) | 74 ± 10 |

## Revendications

1. Composition pour le premier temps d'un procédé de déformation permanente des fibres kératiniques, en particulier des cheveux, caractérisée par le fait qu'elle comprend, dans un support cosmétique approprié, i) au moins un composé de type céramide et ii) au moins un agent réducteur répondant à la formule (I) suivante : 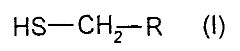 où R désigne un radical carboxy, aminoalkyle en C₁-C₄, carboxyalkyle en C₁-C₄ éventuellement substitué par un groupement amino, uréidoalkyle en C₁-C₄, acyle en C₁-C₄ aminoalkyle en C₁-C₄, aminoacyle en C₁-C₄ aminoalkyle en C₁-C₄, alcoxy en C₁-C₄ carbonyle, monohydroxyalcoxy en C₁-C₄ carbonyle, dihydroxyalcoxy en C₂-C₄ carbonyle, alcoxy en C₁-C₄ carbonyle alkyle en C₁-C₄ éventuellement substitué par un groupement amino, monohydroxy alcoxy en C₁-C₄ carbonyle alkyle en C₁-C₄ éventuellement substitué par un groupement amino, ou dihydroxyalcoxy en C₂-C₄ carbonylalkyle en C₁-C₄ éventuellement substitué par un groupement amino,
ledit composé de type céramide étant présent dans la composition à une teneur allant de 0,005 % à 10 % en poids par rapport au poids total de la composition, ladite composition étant exempte de vésicule contenant un agent réducteur.

2. Composition selon la revendication 1, caractérisée par le fait que ladite teneur va de 0,006 % à 10 %.

3. Composition selon la revendication 2, caractérisée par le fait que ladite teneur va de 0,008 % à 4 %.

4. Composition selon l'une quelconque des revendications 1 à 3, caractérisée par le fait que le composé de type céramide est un composé de formule (II) suivante : 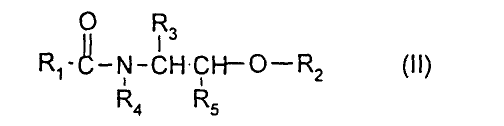 dans laquelle :
- R₁ désigne :
- soit un radical hydrocarboné, linéaire ou ramifié, saturé ou insaturé, en C₁-C₅₀, de préférence en C₅-C₅₀, ce radical pouvant être substitué par un ou plusieurs groupements hydroxyle éventuellement estérifiés par un acide R₇COOH, R₇ étant un radical hydrocarboné, saturé ou insaturé, linéaire ou ramifié, éventuellement mono ou polyhydroxylé, en C₁-C₃₅, le ou les hydroxyles du radical R₇ pouvant être estérifiés par un acide gras saturé ou insaturé, linéaire ou ramifié, éventuellement mono ou polyhydroxylé, en C₁-C₃₅,
- soit un radical R"-(NR-CO)-R', R désigne un atome d'hydrogène ou un radical hydrocarboné C₁-C₂₀ mono ou polyhydroxylé, préférentiellement monohydroxylé, R' et R" sont des radicaux hydrocarbonés dont la somme des atomes de carbone est comprise entre 9 et 30, R' étant un radical divalent,
- soit un radical R₈-O-CO-(CH₂)ₚ, R₈ désignant un radical hydrocarboné en C₁-C₂₀, p étant un entier variant de 1 à 12 ;
- R₂ est choisi parmi un atome d'hydrogène, un radical de type saccharidique, en particulier un radical (glycosyle)ₙ, (galactosyle)ₘ ou sulfogalactosyle, un résidu de sulfate ou de phosphate, un radical phosphoryléthylamine et un radical phosphoryléthylammonium, dans lesquels n est un entier variant de 1 à 4 et m est un entier variant de 1 à 8 ;
- R₃ désigne un atome d'hydrogène ou un radical hydrocarboné en C₁-C₃₃, saturé ou insaturé, hydroxylé ou non, le ou les hydroxyles pouvant être estérifiés par un acide minéral ou un acide R₇COOH, R₇ ayant les mêmes significations que ci-dessus, le ou les hydroxyles pouvant être éthérifiés par un radical (glycosyle)ₙ, (galactosyle)ₘ, sulfogalactosyle, phosphoryléthylamine ou phosphoryléthylammonium, R₃ pouvant également être substitué par un ou plusieurs radicaux alkyle en C₁-C₁₄ ;
- R₄ désigne un atome d'hydrogène, un radical méthyle, éthyle, un radical hydrocarboné en C₃-C₅₀, saturé ou insaturé, linéaire ou ramifié, éventuellement hydroxylé ou un radical -CH₂-CHOH-CH₂-O-R₆ dans lequel R₆ désigne un radical hydrocarboné en C₁₀-C₂₆ ou un radical R₈-O-CO-(CH₂)ₚ, R₈ désigne un radical hydrocarboné en C₁-C₂₀, p étant un entier variant de 1 à 12,
- R₅ désigne un atome d'hydrogène ou un radical hydrocarboné en C₁-C₃₀ saturé ou insaturé, linéaire ou ramifié, éventuellement mono ou polyhydroxylé, le ou les hydroxyles pouvant être éthérifiés par un radical (glycosyle)ₙ, (galactosyle)ₘ, sulfogalactosyle, phosphoryléthylamine ou phosphoryléthylammonium,
sous réserve que lorsque R₃ et R₅ désignent hydrogène ou lorsque R₃ désigne hydrogène et R₅ désigne méthyle alors R₄ ne désigne pas un atome d'hydrogène, un radical méthyle ou éthyle.

5. Composition selon la revendication 4, caractérisée par le fait que le composé de type céramide est un composé de formule (II) pour lesquels R₁ désigne un radical alkyle saturé ou insaturé dérivé d'acides gras en C₁₄-C₂₂ éventuellement hydroxylé; R₂ désigne un atome d'hydrogène ; et R₃ désigne un radical linéaire saturé en C₁₁-C₁₇ éventuellement hydroxylé et de préférence en C₁₃-C₁₅.

6. Composition selon la revendication 5, caractérisée par le fait que le composé de type céramide est choisi parmi :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol,
ou les mélanges de ces composés.

7. Composition selon la revendication 6, caractérisée par le fait que le composé de type céramide est choisi parmi le 2-N-oléoylamino-octadécane-1,3-diol, le 2-N-[2-hydroxy-palmitoyl]-amino-octadécane-1,3-diol et la N-stéaroylphytosphingosine.

8. Composition selon l'une quelconque des revendications précédentes, caractérisée par le fait que l'agent réducteur est choisi dans le groupe formé par l'acide thioglycolique, la cystéamine et la cystéine.

9. Composition selon la revendication 8, caractérisée par le fait que l'agent réducteur est l'acide thioglycolique.

10. Composition selon l'une quelconque des revendications précédentes, caractérisée par le fait que l'agent réducteur est présent dans la composition à une teneur allant de 1 % à 25 % en poids par rapport au poids total de la composition.

11. Composition selon la revendication 10, caractérisée par le fait que ladite teneur va de 3 % à 15 %.

12. Procédé de traitement des matières kératiniques, en particulier des cheveux, en vue d'obtenir une déformation permanente de ces dernières, en particulier sous la forme de cheveux permanentés, ce procédé comprenant les étapes suivantes : (i) on applique sur la matière kératinique à traiter une composition telle que définie à l'une des revendications 1 à 11, la matière kératinique étant mise sous tension mécanique avant, pendant, ou après ladite application, (ii) on rince éventuellement la matière kératinique, (iii) on applique sur la matière kératinique une composition oxydante ou on laisse reposer la matière kératinique éventuellement sous chaleur, (iv) on rince éventuellement à nouveau la matière kératinique.

13. Procédé selon la revendication 12, caractérisé par le fait que la composition oxydante contient un composé de type céramide.

## Patentansprüche

1. Zusammensetzung für den ersten Schritt einer dauerhaften Verformung von Keratinfasern und insbesondere des Haares, die **dadurch gekennzeichnet** ist, daß sie in einem geeigneten kosmetischen Träger (i) mindestens eine Verbindung vom Ceramidtyp und ii) mindestens ein Reduktionsmittel der folgenden Formel (I) enthält:
HS―CH₂―R (I)
wobei R eine Carboxygruppe, eine C₁₋₄-Aminoalkylgruppe, eine C₁₋₄-Carboxyalkylgruppe, die gegebenenfalls mit einer Aminogruppe substituiert ist, eine C₁₋₄-Ureidoalkylgruppe, eine C₁₋₄-Acyl-C₁₋₄-amino-alkylgruppe, eine C₁₋₄-Aminoacyl-C₁₋₄-aminoalkylgruppe, eine C₁₋₄-Alkoxycarbonylgruppe, eine C₁₋₄-Monohydroxyalkoxycarbonylgruppe, eine C₂₋₄-Dihydroxyalkoxycarbonylgruppe, eine C₁₋₄-Alkoxycarbonyl-C₁₋₄-alkylgruppe, die gegebenenfalls mit einer Aminogruppe substituiert ist, eine C₁₋₄-Monohydroxyalkoxycarbonyl-C₁₋₄-alkylgruppe, die gegebenenfalls mit einer Aminogruppe substituiert ist, oder eine C₂₋₄-Dihydroxyalkoxycarbonyl-C₁₋₄-alkylgruppe, die gegebenenfalls mit einer Aminogruppe substituiert ist, bedeutet,
wobei die Verbindung vom Ceramidtyp in der Zusammensetzung in einem Mengenanteil von 0,005 bis 10 Gew.-%, bezogen auf das Gesamtgewicht der Zusammensetzung, vorliegt und die Zusammensetzung keine Vesikel aufweist, die ein Reduktionsmittel enthalten.

2. Zusammensetzung nach Anspruch 1, **dadurch gekennzeichnet**, daß der Mengenanteil im Bereich von 0,006 bis 10 % liegt.

3. Zusammensetzung nach Anspruch 2, **dadurch gekennzeichnet**, daß der Mengenanteil im Bereich von 0,008 bis 4 % liegt.

4. Zusammensetzung nach einem der Ansprüche 1 bis 3, **dadurch gekennzeichnet**, daß die Verbindung vom Ceramidtyp eine Verbindung der folgende Formel (II) ist: 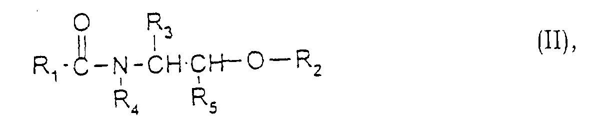 worin bedeuten:
- R₁
- entweder eine geradkettige oder verzweigte, gesättigte oder ungesättigte C₁₋₅₀-Kohlenwasserstoffgruppe und vorzugsweise C₅₋₅₀-Kohlenwasserstoffgruppe, wobei die Gruppe mit einer oder mehreren Hydroxygruppen substituiert sein kann, die gegebenenfalls mit einer Säure R₇COOH verestert sein können, wobei R₇ eine gesättigte oder ungesättigte, geradkettige oder verzweigte C₁₋₃₅-Kohlenwasserstoffgruppe ist, die gegebenenfalls eine oder mehrere Hydroxygruppen aufweist, wobei die Hydroxygruppe(n) der Gruppe R₇ mit einer gesättigten oder ungesättigten, geradkettigen oder verzweigten, gegebenenfalls ein- oder mehrfach hydroxylierten C₁₋₃₅-Fettsäure verestert sein kann (können);
- oder eine Gruppe R"-(NR-CO)-R', wobei R Wasserstoff oder eine C₁₋₂₀-Kohlenwasserstoffgruppe mit einer oder mehreren Hydroxygruppen und vorzugsweise einer Hydroxygruppe bedeutet und R' und R" Kohlenwasserstoffgruppen sind, deren Summe an Kohlenstoffatomen im Bereich von 9 bis 30 liegt, wobei R' eine zweiwertige Gruppe ist,
- oder eine Gruppe R₈-O-CO-(CH₂)ₚ, wobei R₈ eine C₁₋₂₀-Kohlenwasserstoffgruppe ist und p eine ganze Zahl im Bereich von 1 bis 12 bedeutet;
- R₂ Wasserstoff oder eine Gruppe vom Saccharidtyp, insbesondere (Glycosyl)ₙ, (Galactosyl)ₘ oder Sulfogalactosyl, Sulfat, Phosphat, Phosphorylethylamin oder Phosphorylethylammonium, wobei n eine ganze Zahl im Bereich von 1 bis 4 und m eine ganze Zahl im Bereich von 1 bis 8 ist;
- R₃ Wasserstoff oder eine gesättigte oder ungesättigte, gegebenenfalls hydroxylierte C₁₋₃₃-Kohlenwasserstoffgruppe, wobei die Hydroxygruppe(n) mit einer anorganischen Säure oder einer Säure R₇COOH, wobei R₇ die oben angegebenen Bedeutungen aufweist, verestert oder mit einer (Glycosyl)ₙ-Gruppe, (Galactosyl)ₘ-Gruppe, Sulfogalactosylgruppe, Phosphorylethylamingruppe oder einer Phosphorylethylammoniumgruppe verethert sein kann (können), und wobei die Gruppe R₃ auch mit einer oder mehreren C₁₋₁₄-Alkylgruppen substituiert sein kann;
- R₄ Wasserstoff, Methyl, Ethyl, eine gesättigte oder ungesättigte, geradkettige oder verzweigte, gegebenenfalls hydroxylierte C₃₋₅₀-Kohlenwasserstoffgruppe, eine Gruppe -CH₂-CHOH-CH₂-O-R₆, wobei R₆ eine C₁₀₋₂₆-Kohlenwasserstoffgruppe bedeutet, oder eine Gruppe R₈-O-CO-(CH₂)ₚ, wobei R₈ eine C₁₋₂₀-Kohlenwasserstoffgruppe bedeutet und p eine ganze Zahl im Bereich von 1 bis 12 ist;
- R₅ Wasserstoff oder eine gesättigte oder ungesättigte, geradkettige oder verzweigte, gegebenenfalls ein- oder mehrfach hydroxylierte C₁₋₃₀-Kohlenwasserstoffgruppe, wobei die Hydroxygruppe(n) mit einer (Glycosyl)ₙ-Gruppe, (Galactosyl)ₘ-Gruppe, Sulfogalactosylgruppe, Phosphorylethylamingruppe und einer Phosphorylethylammoniumgruppe verethert sein kann (können);
mit der Maßgabe, daß R₄ nicht Wasserstoff, Methyl oder Ethyl bedeutet, wenn R₃ und R₅ Wasserstoff bedeuten oder wenn R₃ Wasserstoff und R₅ Methyl bedeutet.

5. Zusammensetzung nach Anspruch 4, **dadurch gekennzeichnet**, daß die Verbindungen vom Ceramidtyp Verbindungen der Formel (II) sind, worin R₁ eine gesättigte oder ungesättigte, von C₁₄₋₂₂-Fettsäuren abgeleitete, gegebenenfalls hydroxylierte Alkylgruppe, R₂ Wasserstoff und R₃ eine gesättigte, geradkettige, gegebenenfalls hydroxylierte C₁₁₋₁₇-Gruppe und vorzugsweise C₁₃₋₁₅-Gruppe bedeutet.

6. Zusammensetzung nach Anspruch 5, **dadurch gekennzeichnet**, daß die Verbindung vom Ceramidtyp ausgewählt ist unter:
- 2-N-Linoleoylamino-octadecan-1,3-diol,
- 2-N-Oleoylamino-octadecan-1,3-diol,
- 2-N-Palmitoylamino-octadecan-1,3-diol,
- 2-N-Stearoylamino-octadecan-1,3-diol,
- 2-N-Behenoylamino-octadecan-1,3-diol
- 2-N-[2-Hydroxy-palmitoyl]-amino-octadecan-1,3-diol,
- 2-N-Stearoylamino-octadecan-1,3,4-triol und insbesondere N-Stearoylphytosphingosin,
- 2-N-Palmitoylamino-hexadecan-1,3-diol, und den Gemischen dieser Verbindungen.

7. Zusammensetzung nach Anspruch 6, **dadurch gekennzeichnet**, daß die Verbindung vom Ceramidtyp unter 2-N-Oleoylamino-octadecan-1,3-diol, 2-N-[2-Hydroxy-palmitoyl]-amino-octadecan-1,3-diol und N-Stearoylphytosphingosin ausgewählt ist.

8. Zusammensetzung nach einem der vorhergehenden Ansprüche, **dadurch gekennzeichnet**, daß das Reduktionsmittel unter Thioglykolsäure, Cysteamin und Cystein ausgewählt ist.

9. Zusammensetzung nach Anspruch 8, **dadurch gekennzeichnet**, daß das Reduktionsmittel die Thioglykolsäure ist.

10. Zusammensetzung nach einem der vorhergehenden Ansprüche, **dadurch gekennzeichnet**, daß das Reduktionsmittel in der Zusammensetzung in einem Mengenanteil von 1 bis 25 Gew.-%, bezogen auf das Gesamtgewicht der Zusammensetzung, vorliegt.

11. Zusammensetzung nach Anspruch 10, **dadurch gekennzeichnet**, daß der Mengenanteil im Bereich von 3 bis 15 % liegt.

12. Verfahren zur Behandlung von Keratinsubstanzen und insbesondere zur Behandlung der Haare, um ihre dauerhafte Verformung, insbesondere in Form von dauergewelltem Haar, zu erzielen, wobei das Verfahren die folgenden Schritte umfaßt: (i) Auftragen einer Zusammensetzung nach einem der Ansprüche 1 bis 11 auf die zu behandelnden Keratinsubstanzen, wobei die Keratinsubstanzen vor, während oder nach dem Aufbringen der Zusammensetzung unter Spannung gesetzt werden, (ii) gegebenenfalls Spülen der Keratinfasern, (iii) Auftragen einer oxidierenden Zusammensetzung oder Ruhenlassen der Keratinsubstanzen gegebenenfalls unter Wärmeeinwirkung und (iv) gegebenenfalls erneutes Spülen der Keratinfasern.

13. Verfahren nach Anspruch 12, **dadurch gekennzeichnet**, daß die oxidierende Zusammensetzung eine Verbindung vom Ceramidtyp enthält.

## Claims

1. Composition for the first stage of a process of permanent reshaping of keratin fibres, in particular the hair, **characterized in that** it comprises, in a suitable cosmetic support, i) at least one compound of ceramide type and ii) at least one reducing agent corresponding to formula (I) below:
HS-CH₂-R (I)
where R denotes a carboxyl radical, a C₁-C₄ aminoalkyl radical, a carboxy(C₁-C₄)alkyl radical optionally substituted with an amino group, a C₁-C₄ ureidoalkyl radical, a (C₁-C₄)acylamino(C₁-C₄)alkyl radical, an amino(C₁-C₄)acylamino(C₁-C₄)alkyl radical, a (C₁-C₄)-alkoxycarbonyl radical, a monohydroxy(C₁-C₄)-alkoxy-carbonyl radical, a dihydroxy(C₂-C₄)alkoxycarbonyl radical, a (C₁-C₄)alkoxycarbonyl(C₁-C₄)alkyl radical optionally substituted with an amino group, a monohydroxy (C₁-C₄)alkoxycarbonyl (C₁-C₄) alkyl radical optionally substituted with an amino group, or a dihydroxy (C₂-C₄)alkoxycarbonyl(C₁-C₄)alkyl radical optionally substituted with an amino group,
the said compound of ceramide type being present in the composition at a content ranging from 0.005 % to 10 % by weight relative to the total weight of the composition, the said composition being free of vesicles containing a reducing agent.

2. Composition according to Claim 1, **characterized in that** the said content ranges from 0.006 % to 10 %.

3. Composition according to Claim 2, **characterized in that** the said content ranges from 0.008 % to 4 %.

4. Composition according to any one of Claims 1 to 3, **characterized in that** the compound of ceramide type is a compound of formula (II) below: 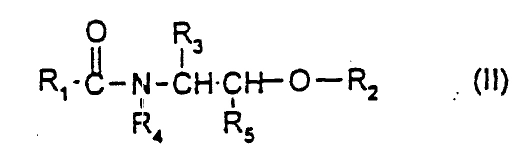 in which:
- R₁ denotes:
- either a saturated or unsaturated, linear or branched C₁-C₅₀, preferably C₅-C₅₀, hydrocarbon radical, it being possible for this radical to be substituted with one or more hydroxyl groups which may be esterified with an acid R₇COOH, R₇ being a saturated or unsaturated, linear or branched, optionally mono- or polyhydroxylated C₁-C₃₅ hydrocarbon radical, it being possible for the hydroxyl(s) of the radical R₇ to be esterified with a saturated or unsaturated, linear or branched, optionally mono- or polyhydroxylated C₁-C₃₅ fatty acid,
- or a radical R"-(NR-CO)-R', R denotes a hydrogen atom or a mono- or polyhydroxylated, preferably monohydroxylated, C₁-C₂₀ hydrocarbon radical, R' and R" are hydrocarbon radicals, the sum of the carbon atoms of which is between 9 and 30, R' being a divalent radical,
- or a radical R₈-O-CO-(CH₂)ₚ, R₈ denoting a C₁-C₂₀ hydrocarbon radical p being an integer ranging from 1 to 12;
- R₂ is chosen from a hydrogen atom, a radical of saccharide type, in particular a (glycosyl)ₙ, (galactosyl)ₘ or sulphogalactosyl radical, a sulphate or phosphate residue, a phosphorylethylamine radical or a phosphorylethylammonium radical, in which n is an integer ranging from 1 to 4 and m is an integer ranging from 1 to 8;
- R₃ denotes a hydrogen atom or a saturated or unsaturated, hydroxylated or non-hydroxylated C₁-C₃₃ hydrocarbon radical, it being possible for the hydroxyl(s) to be esterified with an inorganic acid or an acid R₇COOH, R₇ having the same meanings as above, it being possible for the hydroxyl(s) to be etherified with a (glycosyl)ₙ, (galactosyl)ₘ, sulphogalactosyl, phosphorylethylamine or phosphorylethylammonium radical, it being also possible for R₃ to be substituted with one or more C₁-C₁₄ alkyl radicals;
- R₄ denotes a hydrogen atom, a methyl or ethyl radical, a saturated or unsaturated, linear or branched, optionally hydroxylated C₃-C₅₀ hydrocarbon radical or a radical -CH₂-CHOH-CH₂-O-R₆ in which R₆ denotes a C₁₀-C₂₆ hydrocarbon radical or a radical R₈-O-CO-(CH₂)ₚ, R₈ denotes a C₁-C₂₀ hydrocarbon radical, p being an integer ranging from 1 to 12,
- R₅ denotes a hydrogen atom or a saturated or unsaturated, linear or branched, optionally mono- or polyhydroxylated C₁-C₃₀ hydrocarbon radical, it being possible for the hydroxyl(s) to be etherified with a (glycosyl)ₙ, (galactosyl)ₘ, sulphogalactosyl, phosphorylethylamine or phosphorylethylammonium radical,
with the proviso that when R₃ and R₅ denote hydrogen or when R₃ denotes hydrogen and R₅ denotes methyl, then R₄ does not denote a hydrogen atom or a methyl or ethyl radical.

5. Composition according to Claim 4, **characterized in that** the compound of ceramide type is a compound of formula (II) for which R₁ denotes a saturated or unsaturated, optionally hydroxylated alkyl radical derived from C₁₄-C₂₂ fatty acids; R₂ denotes a hydrogen atom; and R₃ denotes a saturated, linear, optionally hydroxylated C₁₁-C₁₇ and preferably C₁₃-C₁₅ radical.

6. Composition according to Claim 5, **characterized in that** the compound of ceramide type is chosen from:
- 2-N-linoleoylaminooctadecane-1,3-diol,
- 2-N-oleoylaminooctadecane-1,3-diol,
- 2-N-palmitoylaminooctadecane-1,3-diol,
- 2-N-stearoylaminooctadecane-1,3-diol,
- 2-N-behenoylaminooctadecane-1,3-diol,
- 2-N-[2-hydroxypalmitoyl]aminooctadecane-1,3-diol,
- 2-N-stearoylaminooctadecane-1,3,4-triol and in particular N-stearoylphytosphingosine,
- 2-N-palmitoylaminohexadecane-1,3-diol or mixtures of these compounds.

7. Composition according to Claim 6, **characterized in that** the compound of ceramide type is chosen from 2-N-oleoylaminooctadecane-1,3-diol, 2-N-[2-hydroxypalmitoyl]aminooctadecane-1,3-diol and N-stearoylphytosphingosine.

8. Composition according to any one of the preceding claims, **characterized in that** the reducing agent is chosen from the group formed by thioglycolic acid, cysteamine and cysteine.

9. Composition according to Claim 8, **characterized in that** the reducing agent is thioglycolic acid.

10. Composition according to any one of the preceding claims, **characterized in that** the reducing agent is present in the composition at a content ranging from 1 % to 25 % by weight relative to the total weight of the composition.

11. Composition according to Claim 10, **characterized in that** the said content ranges from 3 % to 15 %.

12. Treatment process for keratin substances, in particular the hair, in order to permanently reshape it, in particular in the shape of permanent-waved hair, this process comprising the following steps: (i) a composition as defined in one of Claims 1 to 11 is applied to the keratin substance to be treated, the keratin substance being placed under mechanical tension before, during or after the said application, (ii) the keratin substance is optionally rinsed, (iii) an oxidizing composition is applied to the keratin substance or the keratin substance is optionally left to stand under heat, (iv) the keratin substance is optionally rinsed again.

13. Process according to Claim 12, **characterized in that** the oxidizing composition contains a compound of ceramide type.
